Europäisches Patentamt

European Patent Office

Office européen des brevets

(11) Publication number: **0 256 604 B1**

# EUROPEAN PATENT SPECIFICATION

(45) Date of publication of patent specification: **19.06.91**

(51) Int. Cl.⁵: **C07C 5/27**, C10G 35/04

(21) Application number: **87201533.4**

(22) Date of filing: **12.08.87**

(54) Process for the isomerization of unbranched hydrocarbons.

(30) Priority: **14.08.86 GB 8619784**

(43) Date of publication of application:
**24.02.88 Bulletin 88/08**

(45) Publication of the grant of the patent:
**19.06.91 Bulletin 91/25**

(84) Designated Contracting States:
**AT BE CH DE ES FR GB GR IT LI NL SE**

(56) References cited:
**FR-A- 2 217 298**
**GB-A- 1 315 669**

(73) Proprietor: **SHELL INTERNATIONALE RE-SEARCH MAATSCHAPPIJ B.V.**
**Carel van Bylandtlaan 30**
**NL-2596 HR Den Haag(NL)**

(72) Inventor: **Sie, Swan Tiong**
**Badhuisweg 3**
**NL-1031 CM Amsterdam(NL)**

(74) Representative: **Aalbers, Onno et al**
**P.O. Box 302**
**NL-2501 CH The Hague(NL)**

## Description

The invention relates to a process for the isomerization of unbranched hydrocarbons.

A wide variety of such processes is known, some of which are applied on a commercial scale. However, a problem arises when a feed mixture containing different unbranched hydrocarbons has to be processed because optimal process conditions will be different for the various species of hydrocarbons making up the feed mixture. Such a situation occurs when processing a practical feedstock having a certain boiling range, since hydrocarbons of different molecular mass and boiling point have different reactivities.

It has been proposed to overcome the aforementioned problem by separating a feed mixture into two portions containing lower- and higher-boiling fractions, respectively, and processing said portions separately under isomerization conditions.

A disadvantage of said proposal is, however, that different catalysts are employed for each feed portion and that provisions for product removal and, optionally, for heat exchange, separation and/or recycling of product fractions, have to be present for each feed portion which is separately processed.

It has now been found that the aforementioned disadvantages can be avoided, while maintaining a high degree of flexibility for optimal processing of different unbranched hydrocarbons, by introducing at least two feed fractions having different average molecular masses separately into a series of isomerization zones, provided that each fraction is introduced further downstream in order of increasing molecular mass.

The invention therefore relates to a process for the isomerization of unbranched hydrocarbons which comprises separating a feed mixture containing different unbranched hydrocarbons into at least two fractions having different average molecular masses, introducing each fraction separately in order of increasing molecular mass further downstream into a series of isomerization zones operated at isomerization conditions in the presence of hydrogen and an isomerization catalyst, and removing product from the furthest downstream zone.

The separation of the feed mixture into "lighter" and "heavier" fractions having a lower and higher average molecular mass, respectively, can be carried out in any appropriate manner known in the art e.g. by means of distillation, or by partial evaporation (i.e.flashing) or dephlegmation and gas/liquid separation. The separation according to C-number does not have to be very sharp i.e. molecules having a different number of C-atoms may be present in each fraction.

Various hydrocarbon feed mixtures containing unbranched molecules can be isomerized by means of the process according to the invention, in particular paraffinic feeds, such as $C_5$-$C_6$ paraffinic, hydrocarbons, $C_5$-$C_6$ hydrocarbons containing fractions derived from a crude oil (so-called tops or light naphtha), mixtures of paraffinic hydrocarbons isolated from reformed naphtha (platformate) and light paraffin fractions from the product of a Fischer-Tropsch synthesis. The feed mixture may furthermore contain (un-)saturated cyclic hydrocarbons (e.g. cyclopentane, methylcyclopentane and/or benzene) in quantities of up to 30% by volume without having a substantial negative effect on the present process.

However, in case the feed mixture contains paraffins having 7 or more carbon atoms per molecule in addition to paraffins having 5 and/or 6 carbon atoms per molecule (for which feed mixture the process according to the invention is particularly suited), it is preferred that the various feed fractions substantially contain molecules having the same number of carbon atoms, and accordingly the same molecular mass, in order to avoid cracking of $C_7$ and heavier paraffins present in $C_5$ and/or $C_6$ fractions and to avoid less than optimal isomerization of $C_5$- and/or $C_6$ paraffins present in a predominantly $C_7$ fraction. In this case the average molecular mass of a feed fraction will be approximately equal to the molecular mass of the paraffin present in said fraction.

The average molecular mass as referred to hereinbefore is defined as the total weight divided by the total number of moles of all components present in a particular fraction.

The series of at least two isomerization zones which are required in the process according to the invention in order to attain optimal isomerization of each feed fraction may be located in a single catalyst bed. Thus, in the case of a $C_5$-$C_6$ feedstock the present process is suitably carried out by introducing a lighter fraction containing e.g. substantially n-pentane into the upstream part of the catalyst bed constituting a first zone and introducing a heavier fraction (containing e.g. substantially n-hexane) into a more downstream part of the catalyst bed, i.e., downstream of the above first zone and upstream of the remaining part of the catalyst bed constituting a second zone. In the case of a $C_5$-$C_7$ feedstock, a lighter fraction containing e.g. substantially n-pentane and n-hexane is introduced into the upstream part of the catalyst bed constituting a first zone while a heavier fraction containing, e.g., substantially n-heptane is introduced downstream of the first zone and upstream of a second zone made up by the remainder of the catalyst bed. It is also possible to split the $C_5$-$C_7$ feed in three fractions containing substantially n-pentane, n-hexane and n-heptane, respectively, which fractions are introduced upstream of a first, second and third zone, respectively, of the catalyst bed; the fraction being introduced in a more downstream position as it is heavier.

Preferably, however, a plurality of catalyst beds is employed in the present process, each bed forming a single isomerization zone for which the process conditions can be optimally adjusted to the particular feed fraction introduced therein. The catalyst beds can be suitably incorporated into a single vessel in case two or three beds are applied; alternatively, a plurality of reactors in a series set-up can be applied, each reactor containing one or more catalyst beds.

The isomerization catalysts employed in the isomerization zones are suitably specific heterogeneous hydroisomerization catalysts having an acid activity and a hydrogenation activity and comprising one or more metals from Group VIII of the Periodic Table of the Elements on a carrier material. The carrier material has acidic properties and may suitably consist of silica-alumina, in particular zeolites (e.g. mordenite, faujasite or Y-sieve) in the hydrogen form or exchanged with rare earth ions, or of alumina rendered acidic by combination with halogen (e.g. chlorine). Preferably, the employed catalysts comprise at least one noble metal from Group VIII (in particular platinum) on H-mordenite as carrier material. Most preferably, the H-mordenite is prepared by treating mordenite one or more times with an aqueous solution of an acid (e.g. hydrochloric acid) and, separately, one or more times with an aqueous solution of an ammonium compound (e.g. ammonium nitrate), followed by drying (e.g. at 100-200 °C) and calcining (e.g. at 400-700 °C) of the treated mordenite.

The (noble) metal(s) may be incorporated into the carrier material by any method known in the art, such as impregnation, precipitation or, preferably, ion exchange. If desired, the carrier material can be mixed with an inert binder before or after incorporation of catalytically active metal(s). Alternatively, the metal(s) may be incorporated into the binder before mixing with the carrier material. Suitable binders are e.g. natural clays (such as kaolin or bentonite) and refractory oxides such as alumina, silica, boria, chromia and zirconia or combinations thereof.

The isomerization catalyst particles may have any suitable form, such as tablets, spheres, granules or cylinders. The particles suitably have a diameter from 0.1-10 mm, and preferably from 0.5-5 mm.

Preferably, the fluid space velocity over a zone in the series of isomerization zones is increased in downstream direction in the process according to the invention in order to decrease the severity at which the feed fraction(s) having a higher average molecular mass is (are) processed in the more downstream isomerization zone(s). By introducing heavier feed fractions at downstream points into the main process flow and withdrawing product only from the furthest downstream isomerization zone, said increase in space velocity can be attained in a simple manner. Alternatively, or additionally, the amount of catalyst present in downstream isomerization zones can be varied, e.g., progressively decreased.

The severity with which the heavier feed fraction(s) is (are) isomerized can be further reduced and regulated by operating the present process in such a manner that the temperature decreases in downstream direction in the series of isomerization zones. Such a negative temperature profile over a series of isomerization zones is not normally attained in an isomerization process due to the (usually small) exothermicity of (hydro)-isomerization- and accompanying hydrocracking-reactions; the normal positive temperature profile is disadvantageous in view of the thermodynamic equilibrium limitation of the isomerization reaction.

A negative temperature profile as referred to hereinabove can be suitably attained by introducing a feed fraction into a downstream isomerization zone at a lower temperature than into an upstream zone.

Fresh and/or recycled hydrogen-containing gas is preferably combined with the lightest feed fraction before introduction thereof into the most upstream isomerization zone. In an alternative embodiment of the process according to the invention a hydrogen-containing gas is introduced into a plurality of isomerization zones, in particular into each of said zones; accordingly, the hydrogen to hydrocarbon ratio in each zone can be regulated for optimal isomerization of the particular hydrocarbon mix in that zone. However, the use of separate hydrogen streams makes the process more complicated than in the case of hydrogen introduction exclusively into the most upstream zone. Furthermore a negative temperature profile over the series of said zones can also be obtained by introducing the hydrogen-containing gas into a downstream zone at a lower temperature than into an upstream zone, thus providing further flexibility in operating the present process in an optimal manner.

The molecular hydrogen-containing gas employed in the process according to the invention need not be completely pure and may contain up to 30 mol%, preferably not more than 20 mol%, of other substances such as $CO$, $CO_2$, $N_2$, Argon and/or hydrocarbons, e.g. reformer off-gas, provided that they are substantially inert with respect to the feed and the isomerization catalyst at the isomerization conditions applied.

The process according to the present invention is preferably carried out at temperatures from 100-400 °C, total pressures from 3-100 bar abs., overall space velocities from 0.1-10 kg hydrocarbon feed/l catalyst/hour and overall pure hydrogen/feed molar ratios from 0.1-10. Particularly preferred conditions for isomerization of normal paraffins are temperatures from 240-290 °C, total pressures from 5-50 bar abs., overall space velocities from 0.5-2 kg hydrocarbon feed/l catalyst/hour and overall pure hydrogen/feed molar ratios from 1-5. The overall space velocity is defined as the total weight of hydrocarbons processed per hour divided by the total volume of catalyst in all isomerization zones. The overall hydrogen/feed molar ratio is defined as the total moles of $H_2$ fed to the process over the total moles of hydrocarbons processed, both per unit of time.

The invention further relates to isomerized hydrocarbons whenever prepared according to a process as described hereinbefore.

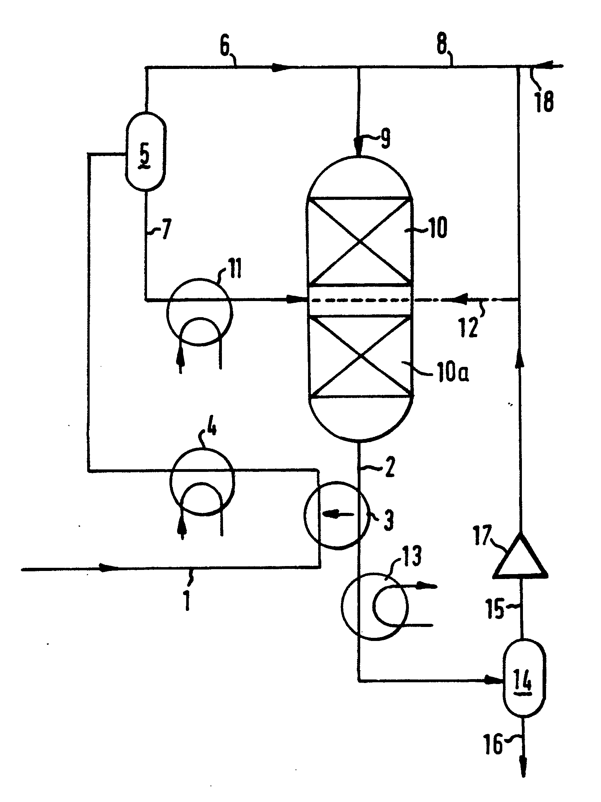

The invention is illustrated with the use of the Figure wherein a specific embodiment is depicted.

A feed stream (1) containing about 60% by weight of n-pentane and 40% by weight of n-hexane is heated by means of heat-exchange with product stream (2) in heat-exchange zone (3), heated further and partially evaporated in heat-exchange zone (4) and separated in separation zone (5) into a first feed fraction substantially containing n-pentane (stream (6)) and a second feed fraction substantially containing n-hexane (stream (7)). Stream (6) is combined with part of the recycled hydrogen-containing gas (stream (8)) into stream (9) before being introduced into the upstream hydroisomerization zone (10) containing a Pt/H-mordenite catalyst, which zone is operated at a temperature of 275 °C and a total pressure of 12 bar. The space velocity in zone (10) is 1.25 kg n-pentane feed/l of catalyst present in zone (10)/hour and the $H_2$/n-pentane feed molar ratio is 3. Stream (7) is passed through heat-exchange zone (11), where it is evaporated and subsequently introduced into downstream hydroisomerization zone (10a) containing a similar catalyst as zone (10). Hydroisomerization zone (10a) is operated at a temperature of 265 °C and a total pressure of 11.5 bar abs. The space velocity in zone (10a) is 5.1 kg $C_5$-$C_6$ hydrocarbons /l of catalyst present in zone (10a)/hour and the $H_2$/$C_5$-$C_6$ hydrocarbons molar ratio therein is 3 which ratio is obtained by adding the remaining part of the recycled hydrogen-containing gas into zone (10a) as stream (12).

Product stream (2) is separated, after condensing and further cooling in heat-exchange zones (3) and (13), in separation zone (14) into a hydrogen-containing gas stream (15) and isomerized product stream (16) which is optionally further separated into branched- and unbranched hydrocarbons, the latter of which may be recycled to at least one hydroisomerization zone (not shown in the Figure). Stream (15) is compressed by means of compressor (17) and combined with fresh hydrogen-containing gas (stream (18)) for making up for the small hydrogen consumption and losses in the process

In the above example, the overall space velocity is 1.3 kg $C_5$-$C_6$ hydrocarbons/l catalyst/hour and the overall $H_2$/$C_5$-$C_6$ hydrocarbon molar ratio 3. The yield of $C_{5+}$ product amounts to 97.4 %w on $C_5$-$C_6$ feed. Of the hydrocarbons in the $C_{5+}$ product, 71.9 % is branched and 28.1 %w consists of unconverted normal paraffins. The amount of $C_4$-hydrocarbons produced is 2.6 %w.

By way of comparison, the same hydrocarbon feed stream was introduced as a single feed into a reactor containing the same amount of catalyst as the total amount in the above example. In this case the reactor was operated at 270 °C and a total pressure of 12 bar. The space velocity was 1.3 kg $C_5$-$C_6$ hydrocarbons/l catalyst/hour and the $H_2$/$C_5$-$C_6$ hydrocarbons molar ratio was 3. The yield of $C_{5+}$ product amounted to 96.5 %w on $C_5$-$C_6$ feed. Of the hydrocarbons in the $C_{5+}$ product, 69.2 %w were branched and 30.8 %w consisted of unconverted normal paraffins. The amount of $C_4$-hydrocarbons produced was 3.5 %w.

**Claims**

1. Process for the isomerization of unbranched hydrocarbons which comprises separating a feed mixture containing different unbranched hydrocarbons into at least two fractions having different average molecular masses, introducing each fraction separately in order of increasing molecular mass further downstream into a series of isomerization zones operated at isomerization conditions in the presence of hydrogen and an isomerization catalyst, and removing product from the furthest downstream zone.

2. Process according to claim 1 wherein the temperature decreases in downstream direction in the series of isomerization zones.

3. Process according to claim 1 or 2 wherein a feed fraction is introduced into a downstream zone at a lower temperature than into an upstream zone.

4. Process according to any one of the preceding claims wherein a hydrogen-containing gas is introduced into a plurality of isomerization zones.

5. Process according to claim 4 wherein the hydrogen-containing gas is introduced into a downstream zone at a lower temperature than into an upstream zone.

6. Process according to any one of the preceding claims wherein the fluid space velocity in the series of isomerization zones is increased in downstream direction.

7. Process according to any one of the preceding claims which is carried out at temperatures from 100-400 °C, total pressures from 3-100 bar, overall space velocities from 0.1-10 kg hydrocarbon feed/l catalyst/hour and overall hydrogen/feed molar ratios from 0.1-10.

8. Process according to any one of the preceding claims wherein the feed mixture contains paraffins having 7 or more carbon atoms per molecule, in addition to paraffins containing 5 and/or 6 carbon atoms per molecule.

## Revendications

1. Procédé pour l'isomérisation d'hydrocarbures non-ramifiés, selon lequel on sépare un mélange de charge contenant des hydrocarbures non-ramifiés différents en au moins deux fractions ayant des poids moléculaires moyens différents, on introduit chaque fraction séparément de plus en plus en aval dans l'ordre des poids moléculaires croissants dans une série de zones d'isomérisation fonctionnant dans des conditions d'isomérisation en présence d'hydrogène et d'un catalyseur d'isomérisation, et on évacue le produit de la zone située le plus en aval.

2. Procédé selon la revendication 1, dans lequel la température s'abaisse en direction aval dans la série des zones d'isomérisation.

3. Procédé selon la revendication 1 ou 2, dans lequel une fraction de charge est introduite dans une zone située en aval à une température plus basse que dans une zone située en amont.

4. Procédé selon l'une quelconque des revendications précédentes, dans lequel un gaz contenant de l'hydrogène est introduit dans une multiplicité de zones d'isomérisation.

5. Procédé selon la revendication 4, dans lequel le gaz contenant de l'hydrogène est introduit dans une zone située en aval à une température plus basse que dans une zone située en amont.

6. Procédé selon l'une quelconque des revendications précédentes, dans lequel la vitesse spatiale fluide dans la série de zones d'isomérisation augmente en direction aval.

7. Procédé selon l'une quelconque des revendications précédentes, qui est mis en oeuvre à des températures de 100 à 400 °C, des pressions totales de 3 à 100 bars, des vitesses spatiales totales de 0,1 à 10 kg de charge d'hydrocarbures par litre de catalyseur et par heure et des rapports molaires d'ensemble hydrogène/charge de 0,1 a 10.

8. Procédé selon l'une quelconque des revendications précédentes, dans lequel le mélange de charge contient des paraffines ayant 7 atomes de carbone ou plus par molécule, en plus de paraffines contenant 5 et/ou 6 atomes de carbone par molécule.

## Ansprüche

1. Verfahren zur Isomerisierung von unverzweigten Kohlenwasserstoffen, welches ein Auftrennen eines vershiedene unverzweigte Kohlenwasserstoffe enthaltenden Einsatzgemisches in wenigstens 2 Fraktionen mit verschiedenen durchschnittlichen Molekularmassen ein getrenntes Einführen jeder Fraktion in der Reihenfolge der zunehmenden Molekularmasse weiter stromabwärts in eine Reihe von Isomerisierungszonen, die bei Isomerisierungsbedingungen in Gegenwart von Wasserstoff und von einem Isomerisierungskatalysator betrieben werden, und ein Abziehen von Produkt aus der am weitesten stromabwärts gelegenen Zone umfaßt.

2. Verfahren nach Anspruch 1, worin die Temperatur in der Reihe von Isomerisierungszonen in stromabwärtiger Richtung abnimmt.

3. Verfahren nach Anspruch 1 oder 2, worin eine Einsatzmaterialfraktion in eine stromabwärtige Zone bei einer niedrigeren Temperatur eingeführt wird als in eine stromaufwärtige Zone.

4. Verfahren nach einem der vorstehenden Ansprüche, worin ein wasserstoffhaltiges Gas in eine Mehrzahl von Isomerisierungszonen eingeführt wird.

5. Verfahren nach Anspruch 4, worin das wasserstoffhaltige Gas in eine stromabwärtige Zone bei einer niedrigeren Temperatur eingeführt wird als in eine stromaufwärtige Zone.

6. Verfahren nach einem der vorstehenden Ansprüche, worin die Fluid-Raumgeschwindigkeit in der Reihe der Isomerisierungszonen in stromabwärtiger Richtung erhöht wird.

7. Verfahren nach einem der vorstehenden Ansprüche, welches bei Temperaturen von 100 bis 400° C, Gesamtdrücken von 3 bis 100 bar, Gesamtraumgeschwindigkeiten von 0,1 bis 10 kg Kohlenwasserstoff-Einsatzmaterial/1 Katalysator/Stunde und Gesamtmolverhältnissen Wasserstoff/Einsatzmaterial von 0,1 bis 10 ausgeführt wird.

8. Verfahren nach einem der vorstehenden Ansprüche, worin das Einsatzmaterialgemisch zusätzlich zu Paraffinen mit einem Gehalt an 5 und/oder 6 Kohlenstoffatomen je Molekül Paraffine mit 7 oder mehr Kohlenstoffatomen je Molekül enthält.